(19) Europäisches Patentamt
European Patent Office
Office européen des brevets

(11) Numéro de publication: **0 193 982**
**A1**

(12) **DEMANDE DE BREVET EUROPEEN**

(21) Numéro de dépôt: **86200237.5**

(22) Date de dépôt: **18.02.86**

(51) Int. Cl.⁴: **C 07 C 127/19**, C 07 C 125/065

(30) Priorité: **01.03.85 FR 8503175**

(43) Date de publication de la demande: **10.09.86 Bulletin 86/37**

(84) Etats contractants désignés: **AT BE DE FR GB IT NL**

(71) Demandeur: **SOLVAY & Cie (Société Anonyme), Rue du Prince Albert, 33, B-1050 Bruxelles (BE)**

(72) Inventeur: **Herman, Jean-Jacques, Drève Richelle, 258, B-1410 Waterloo (BE)**
Inventeur: **Lecloux, André, Van Immersseelbaan,15, B-1860 Meise (BE)**

(54) **Procédé pour la synthèse catalytique de composés organiques azotés, en particulier d'urées et de leurs polymères.**

(57) Procédé pour la synthèse catalytique de composés organiques azotés comprenant la réaction du monoxyde de carbone avec au moins un composé aminé dans un mélange réactionnel comprenant un catalyseur à base de sélénium et une pyridine substituée.

Le procédé convient particulièrement bien à la synthèse d'urées et de leurs polymères.

EP 0 193 982 A1

0193982

# Procédé pour la synthèse catalytique de composés organiques azotés, en particulier d'urées et de leurs polymères

Cas S.84/23

SOLVAY & Cie (Société Anonyme)

La présente invention concerne un procédé pour la synthèse catalytique de composés organiques azotés contenant au moins un groupement > N - CO dans leur molécule. Elle concerne plus particulièrement un procédé pour la synthèse catalytique d'urées et de leurs polymères.

Il est connu de fabriquer des composés organiques azotés,tels que des urées et des carbamates par des méthodes de synthèse impliquant l'insertion catalytique de monoxyde de carbone dans les liaisons >NH - de composés contenant au moins un groupe amino, en présence de catalyseurs métalliques, et dans un milieu réactionnel liquide contenant une base organique azotée.

Ainsi, le brevet GB-A 1275702 (ASAHI KASEI et CHIYODA KAKO) divulgue un procédé de fabrication d'urées ou de dérivés d'urées par réaction d'ammoniac ou d'une amine avec du monoxyde de carbone en présence de sélénium. La réaction est réalisée de préférence en présence d'oxygène et d'un solvant basique tel qu'une triéthylamine ou une amine tertiaire. D'autres solvants basiques, tel que le tétrahydrofurane et la pyridine, ont également été utilisés pour réaliser cette réaction (Journal of American Chemical Society, 1971, volume 93, n° 23, page 6344).

K. Kondo et coll. ont décrit la synthèse de carbamides par réaction d'esters d'acides aminés avec du monoxyde de carbone et de l'oxygène en présence d'un catalyseur au sélénium et de triéthylamine (Synthesis, 1979, n°9, pages 735-736).

La fabrication de carbamates à partir d'amines, d'alcools, de monoxyde de carbone et d'oxygène a été décrite par K.Kondo et coll. dans Chemistry Letters, 1972, n° 5, pages 373-374. Ce document divulgue la mise en oeuvre dans cette réaction d'amines aromatiques primaires, de sélénium et de triéthylamine.

Ces méthodes de synthèse des composés organiques azotés sus mentionnés présentent cependant certains inconvénients. La productivité des réacteurs est faible car les réactions sont trop lentes, malgré la mise en oeuvre de pressions et de températures élevées, ou le rendement des réactions est médiocre, ou la sélectivité en produit final souhaité est mauvaise et on obtient beaucoup de sous-produits indésirables.

La présente invention a pour but de pallier les inconvénients des procédés connus et de fournir un procédé qui donne, avec une bonne productivité, une sélectivité élevée en composé organique azoté de structure souhaitée.

Un but plus particulier de l'invention est de fournir un procédé qui donne, avec un bon rendement, des dérivés et polymères d'urées dont les propriétés sont aisément contrôlables, en particulier des polymères d'urées à haut poids moléculaire.

L'invention concerne à cet effet un procédé pour la synthèse catalytique de composés organiques azotés contenant au moins un groupement > N - CO - dans leur molécule qui comprend la réaction du monoxyde de carbone avec un composé aminé, dans un mélange réactionnel contenant un catalyseur à base de sélénium et un milieu organique liquide, selon lequel le milieu organique liquide contient une pyridine substituée.

Par composés aminés mis en oeuvre dans le procédé selon l'invention, on entend désigner la classe des composés constituée par l'ammoniac et par tous les composés contenant au moins un groupe aminé dont l'azote est mono- ou disubstitué, c'est-à-dire un groupe amino primaire ou secondaire.

Les composés contenant au moins un groupe amino primaire ou secondaire mis en oeuvre dans le procédé selon l'invention répondent généralement à l'une des formules ci-après :

$$R - NH_2 \qquad (I)$$

$$NH_2 - R^1 - NH_2 \qquad (II)$$

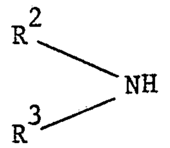 (III)

et 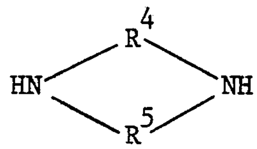 (IV)

Dans les formules (I) et (III) représentant les composés mono-aminés contenant respectivement un groupe amino primaire ou secondaire, R, $R^2$ et $R^3$ représentent des radicaux organiques monovalents quelconques. Ils sont choisis en général parmi les radicaux comprenant de 1 à 20 atomes de carbone. Ces radicaux peuvent être saturés ou insaturés, à chaîne hydrocarbonées ramifiées, droites ou cycliques. Il peut s'agir de radicaux aliphatiques, en particulier de radicaux alkyles ou alkényles comprenant de 1 à 18 atomes de carbone; de radicaux cycloaliphatiques, en particulier de radicaux cycloalkyles comprenant de 5 à 14 atomes de carbone; de radicaux arylaliphatiques, en particulier de radicaux aralkyles comprenant de 7 à 14 atomes de carbone; de radicaux aromatiques monocycliques en particulier de radicaux aryles et alkylaryles contenant de 6 à 14 atomes de carbone et de radicaux aromatiques polycycliques dont les noyaux peuvent être condensés ou réunis par des liens de valence simples, en particulier de radicaux aromatiques à deux noyaux benzéniques.

Les chaînes hydrocarbonées linéaires ou cycliques des radicaux R, $R^2$ et $R^3$ peuvent être interrompues par des hétéroatomes différents du carbone ou par des groupements électrodonneurs quelconques. Les hétéroatomes peuvent être des atomes d'oxygène, de soufre et d'azote, par exemple. Les groupements électrodonneurs peuvent être des groupements sulfoxydés, sulfonés, aminés, amidés, carbonylés, carboxylés et azo, par exemple.

Les radicaux R, $R^2$ et $R^3$ peuvent être substitués par un ou plusieurs substituants quelconques.

Ces substituants peuvent être par exemple eux-mêmes des radicaux

organiques à chaînes hydrocarbonées de même nature que ceux définis ci-dessus. Il peut s'agir également de groupements fonctionnels quelconques, par exemple de groupes halogéno, alkoxy, aryloxy, carboxy, hydroxy, nitro, nitroso, kéto et oxo.

Dans la formule (III), les radicaux $R^2$ et $R^3$ peuvent être identiques ou différents. Ils peuvent être également refermés sur eux-mêmes pour former un cycle dans lequel le groupe amino secondaire est inclus.

Dans la formule (II) représentant les composés diaminés contenant deux groupes amino, $R^1$ représente un radical organique bivalent quelconque. Ce radical, à l'exception de son caractère bivalent, correspond en tous points aux définitions et limitations énoncées ci-avant en rapport avec les radicaux R, $R^2$ et $R^3$. S'il s'agit d'un radical aliphatique, il peut donc être en particulier un radical alkylène ou alkénylène comprenant de 1 à 18 atomes de carbone. S'il s'agir d'un radical cycloaliphatique, il peut être en particulier un radical cycloalkylène comprenant de 5 à 14 atomes de carbone et dans le cas où il s'agit d'un radical aromatique monocyclique, il peut être en particulier un radical arylène ou alkylarylène contenant de 6 à 14 atomes de carbone.

Dans la formule (IV), enfin, $R^4$ et $R^5$, identiques ou différents, représentent également chacun un radical organique bivalent qui est généralement un radical aliphatique et, en particulier, un radical alkylène comprenant de 1 à 10 atomes de carbone.

Des exemples particuliers et non limitatifs de composés aminés répondant à l'une des formules ci-dessus sont :

- les composés ne contenant qu'un groupe amino primaire (formule (I)) comme les monoamines primaires aliphatiques, telles que la méthylamine, l'éthylamine, la n-butylamine et la n-octylamine par exemple; les monoamines primaires cycloaliphatiques, telles que la cyclohexylamine par exemple; les monoamines primaires aromatiques monocycliques, telles que l'aniline et les o-, m- et p-toluidines par exemple; les monoamines primaires alkylaromatiques telles que la benzylamine par exemple; les monoamines primaires aromatiques polycycliques telles que les $\alpha$-et $\beta$- naphtylamines et le 3-aminopyrène par exemple; les

monoamines primaires hétérocycliques telles que la β-aminopyridine et le 2-aminothiazole par exemple; les dérivés alpha monoaminés d'acides carboxyliques aliphatiques et de leurs esters, tels que la glycine, l'alanine, la serine et leurs éthylesters par exemple; les dérivés monoaminés d'acides carboxyliques aromatiques, tels que la proline et l'acide p-aminobenzoïque par exemple, les dérivés monoaminés d'alcools et de phénols tel que la monoéthanolamine et le p-amino phénol par exemple; les dérivés monoaminés d'aldéhydes, tels que la p-aminobenzaldéhyde par exemple; les dérivés monoaminés de quinones, tels que les α- et β- aminoanthraquinones, la 4-amino-1,2-naphtaquinone, la 2-amino-1,4-naphtoquinone par exemple;

- les composés ne contenant qu'un groupe amino secondaire (formule (III)) comme les monoamines secondaires aliphatiques, telles que la diméthylamine, la diéthylamine et la pipéridine par exemple; les monoamines secondaires aromatiques, telles que les dérivés N-substitués de l'aniline, en particulier les dérivés N-alkyl substitués de cette dernière, la diphénylamine et le pyrrole par exemple;
- les composés contenant deux groupes amino primaire (formule (II)) comme les diamines primaires aliphatiques, telles que l'éthylène diamine et la 1,6-hexaméthylène diamine par exemple; les diamines primaires cycloaliphatiques, telles que le 4,4'-diaminodicyclohexyl-méthane par exemple; les diamines primaires aromatiques, telles que la 4,4'-méthylènedianiline, le 4,4'-diaminostilbène, la p-phénylène diamine, le p, p'-diamino diphényle, les 2,4- et 2,6-toluènediamine par exemple; les diamines primaires alkylaromatiques telles que la m-xylylène diamine par exemple; les diamines primaires hétérocycliques, telles que la 2,6-diamino pyridine, par exemple; les dérivés diaminés d'hydrocarbures terpéniques, tels que le 1,8-diamino-p-menthane par exemple; les dérivés diaminés d'acides carboxyliques aliphatiques, tels que la lysine par exemple; les dérivés diaminés d'acides carboxyliques aromatiques, tels que l'acide 3,5-diaminobenzoïque par exemple; les dérivés diaminés de sulfones, tels que la 4,4'-di-aminodicyclohexyl- et la 4,4'-diamino-diphénylsulfones par exemple; les dérivés diaminés d'éthers, tels que le 4,4'-diaminodiphényléther par exemple; les dérivés diaminés de

cétones, tels que la 4,4'-diaminobenzophénone par exemple; les dérivés diaminés de quinones, tels que la 2,6-aminoanthraquinone par exemple;

- les composés contenant deux groupes amino secondaire (formule (IV) comme les diamines secondaires aliphatiques, telles que la pipérazine par exemple.

Le choix du composé aminé à mettre en oeuvre dans le procédé selon l'invention est déterminé par la nature du composé organique azoté que l'on souhaite obtenir. En outre, la nature du composé organique azoté que l'on souhaite obtenir peut nécessiter, de manière connue, la présence éventuelle d'autres réactifs que le composé aminé et le monoxyde de carbone dans le milieu réactionnel.

Ainsi, le procédé selon l'invention permet de fabriquer notamment :

- des urées à partir d'ammoniac ou de composés monoaminés représentés par exemple par l'une des formules (I) ou (II) ci-dessus, de monoxyde de carbone et d'oxygène, par exemple selon l'un des schémas réactionnels ci-après :

$$2NH_3 + CO + 1/2\ O_2 \text{ -----> } NH_2\text{-}CO\text{-}NH_2 + H_2O \qquad (A)$$

$$2RNH_2 + CO + 1/2\ O_2 \text{ -----> } R\text{-}NH\text{-}CO\text{-}NH\text{-}R + H_2O \qquad (B)$$

$$2R^2\text{-}NH\text{-}R^3 + CO + 1/2\ O_2 \text{ -----> } (R^2R^3N)_2CO + H_2O \qquad (C)$$

dans lesquels R, $R^2$ et $R^3$ ont les significations mentionnées plus haut, R étant de préférence choisi parmi les radicaux aliphatiques et aromatiques et $R^2$ et $R^3$ parmi les radicaux aromatiques, plus particulièrement respectivement parmi les radicaux alkyles comprenant de 1 à 10 atomes de carbone, par exemple les radicaux méthyle, éthyle et butyle et parmi les radicaux aryles, par exemple le radical phényle;

- des polyurées à partir de composés diaminés représentés par exemple par la formule (II) ci-dessus, de monoxyde de carbone et d'oxygène, par exemple selon le schéma réactionnel ci-après :

$$(n + 1)(NH_2-R^1-NH_2) + nCO + \frac{n}{2} O_2 \dashrightarrow NH_2-(R^1-NHCONH)_n R^1NH_2 + nH_2O \qquad (D)$$

dans lequel $R^1$ a la signification mentionnée plus haut et est de préférence choisi parmi les radicaux aliphatiques et aromatiques, plus particulièrement respectivement parmi les radicaux alkylène ou alkénylène comprenant de 2 à 10 atomes de carbone et parmi les radicaux arylène ou alkylarylène contenant de 6 à 14 atomes de carbone, et dans lequel n est généralement compris entre 1 et 5000, de préférence entre 2 et 1000;

- des carbamates à partir de composés mono- et diaminés représentés par exemple par les formules (I) et (II) ci-dessus, d'alcools ou de thiols, de monoxyde de carbone et d'oxygène, par exemple selon l'un des schémas réactionnels ci-après :

$$RNH_2 + CO + 1/2\ O_2 + R^6XH \longrightarrow RNH-\overset{\overset{O}{\|}}{C}-XR^6 + H_2O \qquad (E)$$

ou

$$NH_2-R^1-NH_2 + 2CO + O_2 + 2R^6XH \longrightarrow R^6X-\overset{\overset{O}{\|}}{C}-NH-R^1-NH-\overset{\overset{O}{\|}}{C}-XR^6 + 2H_2O \qquad (F)$$

dans lesquels R et $R^1$ ont les significations mentionnées plus haut et sont de préférence des radicaux aromatiques par exemple le radical phényle, $R^6$ est un radical aliphatique, de préférence un radical alkyle linéaire ou branché comprenant de 1 à 10 atomes de carbone par exemple les radicaux méthyle, éthyle ou butyle, et X représente un atome d'oxygène ou de soufre (dans ce dernier cas, le

composé $R^6XH$ est un thiol et le composé organique azoté obtenu est le thiocarbamate correspondant);

- des oligomères d'uréthanes à partir de composés diaminés représentés par exemple par la formule (II) ci-dessus, de monoxyde de carbone, d'oxygène et de diols ou de dithiols, par exemple selon le schéma réactionnel ci-après :

$$nNH_2-R^1-NH_2+2nCO+nO_2+nHX-R^7-XH \dashrightarrow \left[XR^7-X\overset{O}{\overset{\|}{C}}NH-R^1-NH\overset{O}{\overset{\|}{C}}\right]_n+2nH_2O \qquad (G)$$

dans lequel $R^1$ a la signification mentionnée plus haut et est de préférence un radical aromatique par exemple le radical phényle, $R^7$ est un radical aliphatique bivalent, de préférence un radical alkylène comprenant de 1 à 10 atomes de carbone, par exemple le radical méthylène, n est un nombre entier généralement compris entre 1 et 100, de préférence entre 2 et 10, et X représente un atome d'oxygène de soufre.

- des amides à partir des composés mono- et diaminés représentés par exemple par l'une des formules (I) à (IV) ci-dessus et de monoxyde de carbone, par exemple selon l'un des schémas réactionnels ci-après :

$$RNH_2 + CO \dashrightarrow RNH - CO - H \qquad (H)$$

ou

$$NH_2 - R_1 - NH_2 + 2CO \dashrightarrow H - CO - NH - R_1 - NH - CO - H \qquad (I)$$

ou

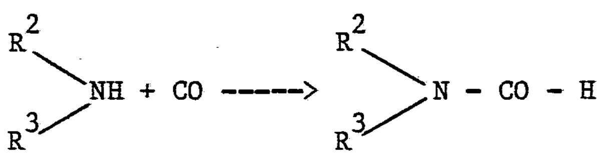

(J)

ou

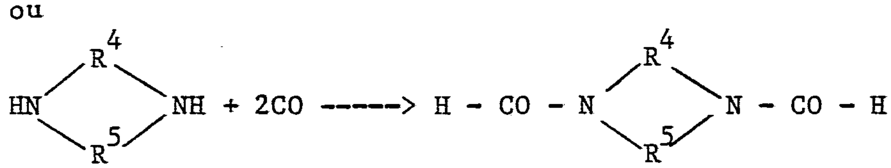

(K)

dans lesquels R, $R^1$, $R^2$, $R^3$, $R^4$ et $R^5$ ont les significations mentionnées plus haut et sont de préférence des radicaux aliphatiques, plus particulièrement des radicaux alkyles ou alkylènes comprenant de 1 à 10 atomes de carbone , par exemple les radicaux méthyle, éthyle, butyle et le radical méthylène.

Le procédé selon l'invention convient particulièrement bien à la synthèse d'urées selon les schémas réactionnels (A) à (C) et de polymères d'urées selon le schéma réactionnel (D) parce qu'il permet l'obtention dans des conditions opératoires particulièrement douces, d'une part d'urées avec une vitesse de réaction et une sélectivité inattendues et, d'autre part, de polyurées de masse moléculaire très élevée.

A titre d'exemples de composés aminés tout particulièrement préférés pour la fabrication de polymères d'urées selon le schéma réactionnel (D), on peut citer la 4,4'-méthylènedianiline , la 2,4-toluènediamine, la 1,6-hexaméthylènediamine, et les p- et m-phénylène diamines. On peut utiliser un seul composé diaminé pour l'obtention d'homopolymères ou plusieurs composés diaminés pour l'obtention de copolymères.

Les conditions opératoires générales dans lesquelles s'effectue la synthèse catalytique des composés organiques azotés selon l'invention sont connues (voir documents cités dans l'introduction de la description dont le contenu est incorporé par référence) et peuvent varier dans une large mesure en fonction de la nature du composé que l'on souhaite fabriquer.

Comme mentionné plus haut, la synthèse est effectuée dans un mélange réactionnel contenant un catalyseur à base de sélénium. Le sélénium présent dans le catalyseur peut être sous forme de sélénium métallique, de sélénium amorphe, ou de composé capable de produire du sélénium dans les conditions de la synthèse. On préfère que le sélénium soit présent sous forme métallique.

La concentration de catalyseur dans le mélange réactionnel peut varier dans une large mesure. En général, elle n'excède pas la quantité que l'on peut dissoudre dans le milieu organique liquide présent dans le mélange, dans les conditions dans lesquelles on

opère la synthèse. Exprimée en mole de sélénium contenu dans le catalyseur par litre de milieu organique, elle est généralement comprise entre 0,005 et 0,5 mole/l, de préférence entre 0,01 et 0,2 mole/l. Les meilleurs résultats ont été enregistrés pour des concentrations en catalyseur comprises entre 0,02 et 0,15 mole de sélénium par litre.

Le mélange réactionnel dans lequel s'effectue la synthèse du composé organique azoté contient un milieu organique liquide. Ce milieu organique liquide est généralement un milieu inerte vis-à-vis des réactifs mis en oeuvre et capable de dissoudre le composé aminé utilisé. Ce milieu organique liquide peut contenir un alcool aliphatique tel que l'éthanol par exemple; une cétone telle que l'acétone et la méthyléthylcétone par exemple; un éther, tel que le dioxane et le tétrahydrofurane par exemple; un hydrocarbure aliphatique tel que l'hexane et le cyclohexane par exemple; un hydrocarbure aromatique tel que le benzène par exemple; une amide N,N disubstituée telle que la diméthylformamide et la diméthylacétamide, par exemple; un ester, tel que l'acétate d'éthyle par exemple; une amine aliphatique tertiaire telle que la triéthylamine par exemple.

Le milieu organique liquide peut contenir un seul des composés ci-dessus ou un mélange de ces composés.

Selon l'invention, le milieu organique liquide contient, éventuellement en association avec au moins un des composés énumérés ci-dessus, une pyridine substituée. On a en effet constaté avec surprise que la présence d'une pyridine substituée dans le milieu organique liquide conduit à une série de résultats avantageux et non prévisibles à priori. Ainsi, cette présence permet de synthétiser des urées, des carbamates, des thiocarbamates, des oligomères d'uréthanes et des amides selon l'un des schémas réactionnels (A) à (C) et (E) à (K) ci-dessus avec des vitesses de réaction élevées, une excellente sélectivité et un très haut rendement dans des conditions douces de température et de pression. En outre, la présence d'une pyridine substituée dans le milieu organique liquide permet, de manière surprenante, de synthétiser des polymères d'urées de masse moléculaire très élevée, dans des conditions de température et de

pression très modérées. C'est ainsi qu'on a pu obtenir, grâce au procédé de l'invention, des polymères d'urées à chaîne linéaire, dont la masse moléculaire moyenne en poids et en nombre (mesurées de manière conventionnelle par chromatographie par perméation de gel) atteignent respectivement 300 . $10^3$ et 70 . $10^3$.

Par pyridine substituée, on entend désigner tous les dérivés de la pyridine portant au moins un substituant quelconque sur le noyau aromatique; ces dérivés peuvent être des dérivés halogénés tels que la 2-chloropyridine, la 3-chloropyridine et la 2,5-chloropyridine par exemple; des dérivés alcoxylés tels que la 3-méthoxypyridine et la 4-méthoxypyridine par exemple; des dérivés alkylés tels que les picolines, les lutidines, les collidines, les éthylpyridines et les propylpyridines par exemple. On préfère utiliser les dérivés dialkylés de la pyridine, et parmi ceux-ci, les lutidines (-2,3, -2,4, -2,5, -2,6, -3,4, -3,5 et -3,6), la 2,4-lutidine (2,4-diméthylpyridine) étant tout particulièrement préférée.

Dans le cas où l'on souhaite assurer une solubilité maximale au composé organique azoté synthétisé selon le procédé de l'invention, on préfère que le milieu organique liquide soit un milieu aussi polaire et basique que possible; on préfère dans ce cas que le milieu organique liquide soit constitué essentiellement du mélange d'une amine aliphatique tertiaire ou d'une amide aliphatique N,N disubstituée avec la pyridine substituée. De bons résultats, notamment dans le cas où la synthèse de polyurées à haut poids moléculaire ont été obtenus dans un milieu organique liquide comprenant un mélange de diméthylacétamide ou de diméthylformamide et de 2,4-lutidine, dans des proportions molaires entre l'amide et la lutidine généralement comprises entre 1/10 et 10/1, de préférence entre 1/1 et 4/1.

Selon l'invention, le milieu organique liquide peut contenir en outre un halogénure de métal alcalin, de préférence un halogénure de lithium, plus particulièrement un chlorure ou un bromure de ce métal, qui permet d'augmenter la solubilité du composé organique azoté dans le mélange réactionnel; ceci se révèle particulièrement avantageux dans le cas de la synthèse de polyurées à haut poids

moléculaire. Habituellement, l'halogénure de lithium est présent dans le milieu organique liquide à raison de 0,1 à 10 moles par mole de pyridine substituée, de préférence à raison de 1 à 5 moles par mole. Les meilleurs résultats ont été obtenus lorsque le milieu organique liquide comprend un mélange de diméthylacétamide et de 2,4-lutidine auquel on a ajouté environ 1 mole d'halogénure de lithium par mole de 2,4-lutidine.

La concentration du composé aminé dans le mélange réactionnel peut varier dans de larges limites. Généralement, la limite supérieure de concentration en composé aminé du mélange réactionnel est conditionnée par la viscosité conférée à ce dernier. Elle est habituellement comprise entre 0,01 et 5 moles de composé aminé par litre de volume de mélange réactionnel, de préférence entre 0,1 et I mole par litre.

Les autres réactifs nécessaires à la synthèse du composé organique azoté souhaité sont introduits dans le mélange réactionnel dans des rapports molaires, vis-à-vis du composé aminé, qui sont de l'ordre de grandeur requis par la schéma réactionnel conduisant audit composé organique.

L'ordre d'introduction des réactifs dans le mélange réactionnel est quelconque. On préfère en général introduire le monoxyde de carbone -sous forme pure ou sous forme d'un gaz le contenant- dans le mélange réactionnel dans lequel ont été préalablement introduits le composé aminé, le catalyseur et les éventuels autres réactifs et, simultanément ou, de préférence, ultérieurement à l'introduction de monoxyde de carbone, introduire lentement l'oxygène.

La température et la pression auxquelles on opère la réaction peuvent varier dans de très larges limites. Le procédé de l'invention permet toutefois d'opérer dans des conditions de température et de pression inférieures à celles utilisées dans les procédés de synthèse de l'art antérieur. Elles sont choisies en fonction de la nature du composé organique azoté que l'on souhaite synthétiser et de manière à ne pas dépasser la température de décomposition du mélange réactionnel. La température est habituellement inférieure à 250°C. Elle est généralement comprise entre 150 et 200°C dans le cas des synthèses représentées par les schémas réactionnels (E) et (K). De manière surprenante, le procédé selon l'invention permet d'effectuer les synthèses représentées par les schémas réactionnels (A) à (D), en particulier la synthèse des polyurées selon le schéma (D), à des températures remarquablement basses, habituellement comprises entre 0 et 70°C, de préférence entre 20 et 40°C.

La pression totale est habituellement inférieure à 10 MPa; elle est de préférence comprise entre 2 et 5 MPa.

La durée de la réaction dépend de la nature du composé organique azoté que l'on souhaite synthétiser. Elle peut varier de quelques minutes à plusieurs dizaines d'heures. Grâce au procédé selon l'invention, les vitesses de réaction sont en général très élevées, permettant par exemple la synthèse de carbamates et d'urées en quelques (20 à 30) minutes. Des polyurées de viscosité intrinsèque très élevée (jusqu'à 0,43 l/g environ mesurée à 25°C dans la diméthylformamide molaire en LiBr) correspondant aux valeurs de masses moléculaires en poids et en nombre mentionnées plus haut peuvent être obtenues en quelques (2 à 3) heures.

Le procédé selon l'invention peut être mis en oeuvre en continu ou en discontinu dans un réacteur unique ou dans des réacteurs en parallèle ou en série. Pour réaliser le procédé selon l'invention, on peut utiliser n'importe quel appareillage convenant pour les mélanges réactionnels liquides.

Les exemples qui suivent illustrent l'invention sans en limiter la portée.

<u>Exemple 1</u>

Dans un autoclave en acier inoxydable de 0,3 l chauffé par un four électrique, muni d'un agitateur, d'un serpentin pour la circulation d'eau de réfrigération, d'un thermocouple servant à la lecture et à la régulation de température et d'un tube plongeant pour l'introduction des gaz, on introduit 0,63 g de sélénium métallique (8 mmoles) et 90 ml d'une solution C.

La solution C est préparée de la manière suivante :

(a) on prépare au préalable 1 litre de solution A de diméthylacétamide 2,12 molaire en chlorure de lithium;

(b) on prépare ensuite un litre de solution B contenant un mélange

de la solution A et de 2,4-diméthylpyridine (2,4-lutidine), de telle sorte que la solution B soit 2,39 molaire en 2,4-lutidine;
(c) on prépare enfin 1 litre de solution C en dissolvant 115,7 g de 4.4'-méthylènedianiline (MDA) dans la solution B, de telle sorte que la solution C soit 0,58 molaire en MDA.

Dans l'autoclave ensuite fermé ramené à la pression atmosphérique et dont le contenu est amené à 30°C, on introduit du monoxyde de carbone en quantité nécessaire à l'obtention du rapport molaire CO/MDA=3.

24 minutes après le début de l'introduction du CO, on introduit l'oxygène à un débit de 0,2 l/min et en quantité nécessaire pour atteindre le rapport molaire $O_2$/MDA=1.5.

La réaction est poursuivie sous pression autogène pendant 2 heures en maintenant la température à 30°C; l'autoclave est ensuite ramené à la température ambiante, dégazé et son contenu recueilli.

Après avoir éliminé le sélénium par barbotage d'air et filtration, le milieu réactionnel est versé dans 10 fois son volume de méthanol pour précipiter le polymère formé.

Le polymère est récupéré par filtration, lavé au méthanol et à l'acétone, puis séché à 70°C. Le rendement est pratiquement quantitatif.

Le polymère d'urée obtenu est composé d'enchaînements macromoléculaires linéaires comme le montre leur examen par RMN ($^{13}C$).

La viscosité intrinsèque du polymère mesurée à 25°C dans la diméthylformamide molaire en bromure de lithium vaut 0,382 l/g.

Exemples 2 et 3

Ces exemples sont donnés à titre de comparaison.

On reproduit exactement le mode opératoire de l'exemple 1 sauf que dans la préparation de la solution B, on remplace la 2,4-diméthylpyridine par une même quantité molaire de triéthylamine (exemple 2) ou de pyridine (exemple 3).

Les viscosités intrinsèques des polyurées obtenues, mesurées comme indiqué à l'exemple 1 sont mentionnés dans le tableau ci-après :

TABLEAU

| | Nature de la base | Viscosité intrinsèque du polymère (l/g) |
|---|---|---|
| Exemple 2 | triéthylamine | 0,033 |
| Exemple 3 | pyridine | 0,035 |

La comparaison de ces résultats avec ceux de l'exemple 1 montre que la présence de 2,4-lutidine dans le milieu organique liquide permet l'obtention, toutes autres conditions égales, de polyurées de masse moléculaire moyenne nettement plus élevée.

Exemple 4

Dans un autoclave tel que celui utilisé à l'exemple 1, on ajoute 10 ml d'aniline (109,7 m moles) à une solution contenant 75 ml de 2,4-lutidine, 35 ml de méthanol et 0,3 g de sélénium. Lorsque l'on a atteint la température de 170°C, on introduit successivement du monoxyde de carbone à la pression partielle de 4 MPa et de l'oxygène à la pression partielle de 1,5 MPa. La pression totale dans l'autoclave est maintenue constante pendant toute la durée de la réaction en laissant la bonbonne d'oxygène ouverte. Après 9 minutes à 170°C, l'analyse montre que l'on a consommé 106,4 mmoles (taux de transformation de 97 %) pour former 97,9 mmoles de N-phényl-carbamate de méthyle (sélectivité de 92 %).

Exemple 5

Cet exemple est donne à titre de comparaison. On procède de la même manière qu'à l'exemple 4 sauf que l'on remplace la 2,4-lutidine par un volume équivalent de pyridine. Après 9 minutes de réaction à 170°C, le taux de transformation était de 96 % et la sélectivité en carbamate de 63 % seulement.

Exemple 6

Dans un autoclave de 5 l similaire à celui utilisé à l'exemple 1, on introduit 85 ml d'aniline, 1, 16 l de diméthylformamide, 0,44 l de 2,4 lutidine et 2,8 g de sélénium.

L'autoclave est fermé et le milieu amené à 30°C. On introduit le monoxyde de carbone en quantité nécessaire à l'obtention du rapport molaire CO/aniline=3. 24 minutes après l'introduction du monoxyde de carbone, on introduit l'oxygène au débit de 0,6 l/min. et en quantité nécessaire pour atteindre le rapport molaire $O_2$/aniline=1,5.

La réaction est poursuivie sous pression autogène pendant 1 heure en maintenant la température à 30°C. L'autoclave est ensuite dégagé et son contenu recueilli. L'analyse du milieu réactionnel par chromatographie en phase vapeur indique une conversion totale de l'aniline en diphénylurée.

Exemple 7

Dans un autoclave de 0,5 l similaire à celui décrit dans l'exemple 1, on introduit 0,5 gr d'oxyde de sélénium ($SeO_2$), 75 ml de 2,4-lutidine et 7,66 gr de $NH_3$ et on porte le milieu réactionnel à 30°C. On introduit alors 11,09 litres normaux de CO puis 7.39 litres normaux d'oxygène à un débit de 0.39 litres normaux/min. 30 minutes après le début de l'introduction de l'oxygène, l'autoclave est refroidi et purgé. Le rendement en urée est supérieur à 86 %.

Exemple 8

Cet exemple est donné à titre de comparaison.

L'exemple 7 est reproduit mais en remplaçant la 2,4-lutidine par un volume équivalent de méthanol; la quantité de $NH_3$ introduite est de 7,70 g. Le rendement en urée est de 5 %.

## REVENDICATIONS

1 - Procédé pour la synthèse catalytique de composés organiques azotés contenant au moins un groupement > N - CO - dans leur molécule, comprenant la réaction du monoxyde de carbone avec au moins un composé aminé dans un mélange réactionnel comprenant un catalyseur à base de sélénium et un milieu organique liquide, caractérisé en ce que le milieu organique liquide contient une pyridine substituée.

2 - Procédé selon la revendication 1, appliqué à la synthèse catalytique d'urées, caractérisé en ce que le composé aminé est choisi parmi l'ammoniac et les composés monoaminés et en ce que le mélange réactionnel contient en outre de l'oxygène.

3 - Procédé selon la revendication 1, appliqué à la synthèse catalytique de polyurées, caractérisé en ce que le composé aminé est au moins un composé diaminé et en ce que le mélange réactionnel contient en outre de l'oxygène.

4 - Procédé selon la revendication 1 appliqué à la synthèse catalytique de carbamates, caractérisé en ce que le composé aminé est choisi parmi les composés mono- et diaminés, et en ce que le mélange réactionnel contient en outre de l'oxygène et un alcool.

5 - Procédé selon la revendication 1, appliqué à la synthèse catalytique d'oligomères d'uréthanes, caractérisé en ce que le composé aminé est choisi parmi les composés diaminés et en ce que le mélange réactionnel contient en outre de l'oxygène et un diol.

6 - Procédé selon la revendication 1, appliqué à la synthèse catalytique d'amides, caractérisé en ce que le composé aminé est choisi parmi les composés mono- et diaminés.

7 - Procédé selon l'une quelconque des revendications 1 à 6, caractérisé en ce que le milieu organique liquide contient, outre la pyridine substituée, une amide aliphatique N,N-disubstituée.

8 - Procédé selon la revendication 7, caractérisé en ce que le milieu organique liquide contient en outre un halogénure de lithium.

9 - Procédé selon l'une quelconque des revendications 1 à 8, caractérisé en ce que la pyridine substituée est un dérivé alkylé de la pyridine.

10 - Procédé selon la revendication 9, caractérisé en ce que la pyridine substituée est la 2,4-lutidine.

Office européen des brevets

0193982

Numero de la demande

## RAPPORT DE RECHERCHE EUROPEENNE

EP 86 20 0237

**DOCUMENTS CONSIDERES COMME PERTINENTS**

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 036 895 (BASF)<br>* Revendications; page 10 *<br>----- | 1-10 | C 07 C 127/19<br>C 07 C 125/065 |
| | | | DOMAINES TECHNIQUES RECHERCHES (Int. Cl.4) |
| | | | C 07 C 127/00<br>C 07 C 125/00 |

Le présent rapport de recherche a été établi pour toutes les revendications

| Lieu de la recherche | Date d'achèvement de la recherche | Examinateur |
|---|---|---|
| LA HAYE | 09-06-1986 | GAUTIER R.H.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82